(19) Europäisches Patentamt / European Patent Office / Office européen des brevets

(11) **EP 4 016 470 A1**

(12) **EUROPEAN PATENT APPLICATION**

(43) Date of publication:
**22.06.2022 Bulletin 2022/25**

(21) Application number: **21214467.9**

(22) Date of filing: **14.12.2021**

(51) International Patent Classification (IPC):
**G06V 10/44** (2022.01)

(52) Cooperative Patent Classification (CPC):
**G06V 10/44; G06V 2201/03**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.12.2020 KR 20200177192**

(71) Applicants:
• **Industry-Academic Cooperation Foundation, Yonsei University**
  **Seoul 03722 (KR)**

• **Institute for Basic Science**
  **Yuseong-gu**
  **Daejeon 34126 (KR)**

(72) Inventors:
• **Lee, Sang Hwy**
  **07981 Seoul (KR)**
• **Kang, Sung Ho**
  **30062, Sejong-si (KR)**
• **Jeon, Ki Wan**
  **34049 Daejeon (KR)**

(74) Representative: **RGTH**
  **Patentanwälte PartGmbB**
  **Neuer Wall 10**
  **20354 Hamburg (DE)**

(54) **3D MORHPHOLOGICAL OR ANATOMICAL LANDMARK DETECTION METHOD AND DEVICE USING DEEP REINFORCEMENT LEARNING**

(57) Provided are 3D morphological or anatomical landmark detection method and device using deep reinforcement learning, in which the method includes performing volume rendering on 3D image data to generate 3D volume rendered model data, and applying a single-stage deep reinforcement learning (DRL), including obtaining a 2D coordinate value corresponding to a landmark point on a 2D view image, which is constructed by the 3D volume rendered model data, by using a deep reinforcement learning agent, and obtaining a remaining 1D coordinate value corresponding to the landmark point based on an 1D profile of the 3D image data which is obtained based on the 2D coordinate value. The method may further include applying a multi-stage DRL, including detecting the landmark point by repeating the operations of obtaining the 2D coordinate value corresponding to the landmark point on the 2D view image, which is constructed by the 3D volume rendered model data, by using the deep reinforcement learning agent, and, from a 2D view image which is determined based on the obtained 2D coordinate value, obtaining a 2D coordinate value corresponding to the landmark point by using a deep reinforcement learning agent.

**Description**

TECHNICAL FIELD

The present invention relates to 3D morphological or anatomical landmark detection method and device, and more particularly, to method and device capable of accurately detecting 3D morphological or anatomical landmark based on deep reinforcement learning and volume rendering imaging.

BACKGROUND

In order to understand and apply the morphological characteristics of various morphological or anatomical structures of human body or animal, it is necessary to find landmarks and analyze the relationship between them. Therefore, finding morphological or anatomical landmarks is essential in many biological researches and their applications, but it is difficult to find the landmarks in many target samples. Numerous attempts have been made to automate this process, and effort is made to propose a method of acquiring landmark using artificial intelligence.

The complexity and importance of the head would be greater than any part of human body. 3D cephalometry uses computed tomography (CT) images for measuring craniofacial morphology, and has been applied in various medical and biological fields in order to conduct treatments and/or researches on craniofacial deformity, clinical dentistry, growth, anthropology and comparative anatomy. The cephalometry of 2D images has long played a central role in these applications. The advantages associated with accurate anatomical structure identification and complex facial structure evaluation according to recent scientific and technological developments have led to rapid adoption of 3D cephalometric analysis. However, despite these surprising advantages, the manual detection of landmarks on 3D data requires considerable time and expertise, and this remains a major obstacle to the widespread adoption of 3D cephalometric analysis.

Various machine learning algorithms for 3D automatic cephalometric landmark detection show remarkable performance when compared to conventional models or knowledge-based approaches. As a result of reviewing recent 3D automatic cephalometric landmark detection methods, it has been reported that the machine or deep learning method produced the excellent results.

Among various machine learning methods, deep reinforcement learning (DRL) has recently been attracting attention for its excellence in 3D localization. DRL is also considered useful for limited label data tasks, because it is frequently used in medical research involving normal or diseased human models.

In the course of clinical performance of cephalometric analysis and self-assessment of 3D cephalometric studies, it was found that landmark detection by experts/clinicians tend to share common patterns. First, based on the anatomical knowledge and the characteristic direction of the radiographic image, the global features of the image are focused and then, for the final determination of the coordinate values of the landmark, the focus after capture of the global feature shifts to the local region. This pattern of shifting interest from global to local is well known and has been applied to automatic cephalometry and to 2D cephalometry, in particular.

However, since annotation of 3D landmarks in 3D models increases the complexity and dimension of anatomical structures compared to that in 2D models, it cannot be completed simply using this approach.

SUMMARY

An object of the present invention is to provide 3D morphological or anatomical landmark detection method and device using deep reinforcement learning.

A 3D landmark detection method according to an embodiment of the present invention may include the operations of: performing volume rendering on 3D image data to generate 3D volume rendered model data; applying a single-stage deep reinforcement learning (DRL), and/or applying a multi-stage DRL. The operation of applying the single-stage DRL may include the operations of: obtaining a 2D coordinate value corresponding to a landmark point on a 2D view image, which is constructed by the 3D volume rendered model data, by using a deep reinforcement learning agent; and obtaining a remaining 1D coordinate value corresponding to the landmark point based on an one-dimensional (1D) profile of the 3D image data which is obtained based on the 2D coordinate value. The 1D profile may be constructed of a 3D image data value on an axis perpendicular to the 2D view image and passing through a point that corresponds to the 2D coordinate value. The operation of applying a multi-stage DRL may include detecting the landmark point by repeating the operations of: obtaining the 2D coordinate value corresponding to the landmark point on the 2D view image, which is constructed by the 3D volume rendered model data, by using the deep reinforcement learning agent; and, from a 2D view image which is determined based on the obtained 2D coordinate value, obtaining a 2D coordinate value corresponding to the landmark point by using a deep reinforcement learning agent.

The operation of applying the single-stage DRL or the operation of applying the multi-stage DRL may be selectively employed according to characteristics of the landmark point.

Meanwhile, the deep reinforcement learning agent may be trained with a deep reinforcement learning algorithm to detect a feature point corresponding to the landmark point on the 2D view image which is constructed by the 3D volume rendered model data.

The operation of applying the single-stage DRL may include the operations of: obtaining the 2D coordinate value corresponding to the landmark point on the 2D view image, which is constructed by the 3D volume rendered model data, through the deep reinforcement learning agent; and applying a nonlinear diffusion equation to the 1D profile of the 3D image data corresponding to the remaining 1D coordinate value, and then obtaining the remaining one coordinate value based on a position with a maximum value of a first order derivative.

The operation of applying the single-stage DRL may include the operations of: obtaining the 2D coordinate value corresponding to the landmark point on the 2D view image, which is constructed by the 3D volume rendered model data, through the deep reinforcement learning agent; transforming the 1D profile of the 3D image data corresponding to the remaining 1D coordinate value by using the following equation; applying a nonlinear spreading profile to remove noise from the transformed profile; and taking the first order derivative of the profile to which the nonlinear diffusion equation is applied, and then obtaining the remaining 1D coordinate value based on the position with the maximum slope,

$$IE(x) = \tanh\left(\frac{x - L}{W}\right)$$

where x may be the 3D image data value, L may be a center of a 3D image data value range, and W may be a scale value.

The applying the multi-stage DRL may further include obtaining a first 2D coordinate value corresponding to the landmark point on a 2D projection view image, which is constructed by the 3D volume rendered model data, by using a first deep reinforcement learning agent, and obtaining a second 2D coordinate value corresponding to the landmark point on a first cut view image, which is constructed by the 3D volume rendered model data, by using a second deep reinforcement learning agent, in which the first cut view image may correspond to a plane defined by the first 2D coordinate value.

It is possible to obtain a 3D coordinate value corresponding to the landmark point by using a coordinate value among the first 2D coordinate values which is not included in the second 2D coordinate value, and the second 2D coordinate value.

The applying the multi-stage DRL may further include obtaining a third 2D coordinate value corresponding to the landmark point on a second cut view image, which is constructed by the 3D volume rendered model data, by using a third deep reinforcement learning agent, in which the second cut view image may correspond to a plane defined by the second 2D coordinate value.

The 3D image data may be any one of Computed Tomography (CT), Cone Beam Computed Tomography (CBCT), Magnetic Resonance Imaging (MRI), Positron Emission Tomography (PET), and Single-Photon Emission Computed Tomography (SPECT).

The type of landmark may be previously determined.

The 3D image data may be acquired by capturing a patient's skull or other parts of a body.

According to the present invention, there is an advantage in that the 3D landmark can be efficiently and accurately detected according to the type of 3D landmark, while overcoming the complexity of 3D image data processing.

BRIEF DESCRIPTION OF THE DRAWING

FIG. 1 is a block diagram of a 3D anatomical landmark detection device using multi-stage deep reinforcement learning according to an embodiment of the present invention.
FIG. 2 is a diagram conceptually illustrating the operation of the 3D anatomical landmark detection device using multi-stage deep reinforcement learning according to an embodiment of the present invention.
FIG. 3 is a diagram provided to explain a first order derivative-based boundary estimation technique according to an embodiment of the present invention.
FIG. 4 is a flowchart illustrating a 3D anatomical landmark detection method using multi-stage deep reinforcement learning according to an embodiment of the present invention.

DETAILED DESCRIPTION

The exemplary embodiments of the present invention will be described in detail below with reference to the accompanying drawings for those of ordinary skill in the art to be able to easily achieve the present invention.

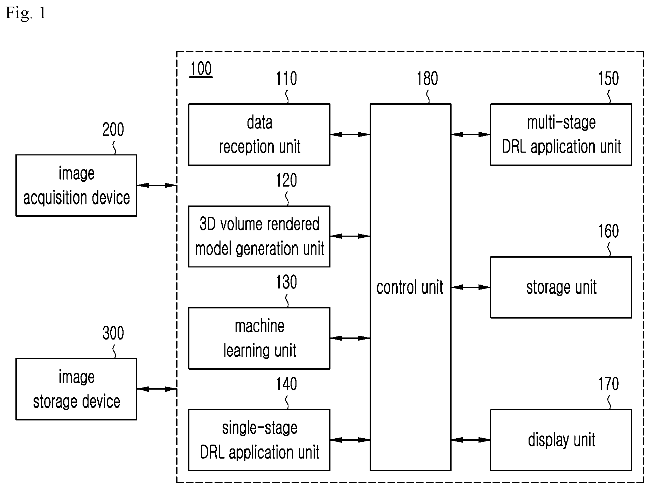

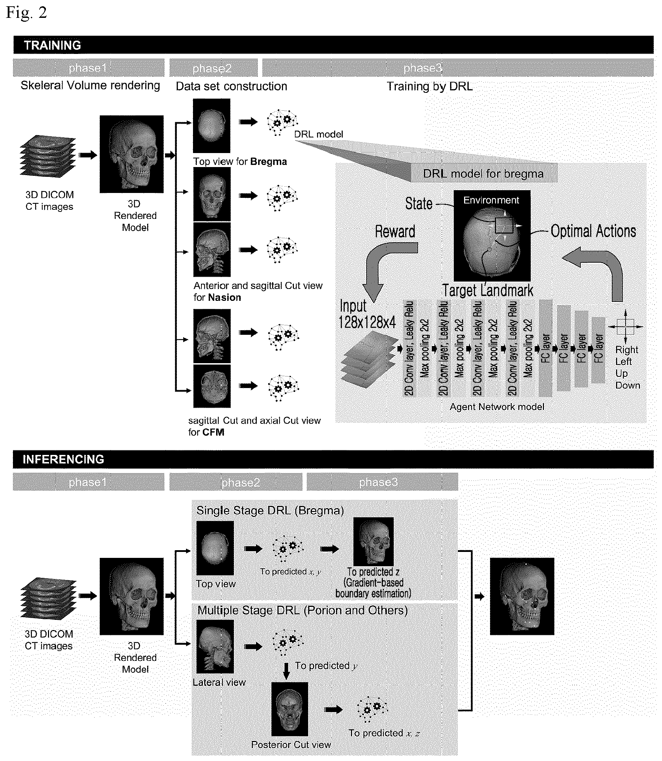

FIG. 1 is a block diagram of a 3D anatomical landmark detection device using multi-stage deep reinforcement learning according to an embodiment of the present invention, and FIG. 2 is a diagram conceptually illustrating the operation of the 3D anatomical landmark detection device using multi-stage deep reinforcement learning according to an embodiment of the present invention.

Referring to FIG. 1, a 3D landmark detection system according to the present invention may include an image acquisition device 200, an image storage device 300, and a 3D landmark detection device 100.

The image acquisition device 200 is a device for acquiring three dimension (3D) image data of an object. In this example, the 3D image data may be data acquired in the form of Computed Tomography (CT), Cone Beam Computed Tomography (CBCT), Magnetic Resonance Imaging (MRI), Positron Emission Tomography (PET), Single-Photon Emission Computed Tomography (SPECT), and the like. The object may be a body part such as a skull and the like.

The image storage device 300 may store 3D image data acquired by the image acquisition device 200 or provided from an external medical image information providing institution including an external medical institution or research institution.

The 3D landmark detection device 100 may detect 3D landmark points from 3D image data provided from the image acquisition device 200 or the image storage device 300.

To this end, the 3D landmark detection device 100 includes a data reception unit 110, a 3D volume rendered model generation unit 120, a machine learning unit 130, a single-stage DRL application unit 140, a multi-stage DRL application unit 150, a storage unit 160, a display unit 170, and a control unit 180.

The data reception unit 110 may receive 3D image data from the image acquisition device 200 or the image storage device 300. For example, the data reception unit 110 may receive DICOM format CT data from the image acquisition device 200 or the image storage device 300.

The 3D volume rendered model generation unit 120 may perform volume rendering on 3D image data to generate 3D volume rendered model data. For example, the 3D volume rendered model generation unit 120 may generate volume rendered 3D model data by using 3D visualization software library according to the following steps. A 2D projection image is acquired by volume rendering algorithm, and a volume rendering method specifically emphasizing bones may be applied.

This volume rendering technique is a well-known technique for visualizing the 3D spatial dimension of a sampled function by computing a 2D projection of a color translucent volume. The volume rendering visualization of 3D CT data helps to recognize anatomy more clearly and to move easily between bone and soft tissue profiles. The latter function is a frequently performed procedure in 2D and 3D head measurements and may be useful in detecting bone and soft tissue landmarks.

The volume rendered 3D model data may be constructed into the 2D view images. The 2D view images may include 2D projection view images having a view direction toward the front (anterior view), toward the back (posterior view), from above (top view), from below (bottom view), toward the left (left view), toward the right (right view), and the like. In addition, the volume rendered 3D model data may include a plurality of 2D cut views. In this case, the 2D cut view may be a view that is selectively removed to visualize internal features without completely sacrificing external context. The 3D volume rendered model generation unit 120 may perform voxel preprocessing by applying transparency to an uninterested region in order to find the landmark points in the cut view.

The machine learning unit 130 may train a deep reinforcement learning agent with deep reinforcement learning algorithm to detect 2D feature points corresponding to the landmark points in the 2D view image.

As exemplified in TRAINING phase 3 in FIG. 2, the deep reinforcement learning training is configured in such a way that the environment responds to the agent's actions, and the agent continuously acts to obtain the maximum reward from the environment, that is, to reach a position closest to the reference coordinates. For the deep reinforcement learning algorithm, the reinforcement learning training framework known as Double Deep Q-Network may be adopted.

The deep reinforcement learning agent may be trained for each 2D view image with respect to each landmark point. As illustrated in TRAINING phase 2 in FIG. 2, the deep reinforcement learning agent may be trained using a top view image for bregma. In addition, for nasion, the deep reinforcement learning agent may be trained using an anterior view image and a sagittal cut view image, respectively. Meanwhile, for the center of foramen magnum (CFM), the deep reinforcement learning agent may be trained using a sagittal cut view image and an axial cut view image, that is, with respect to two cut view images, respectively.

For each landmark, it may be previously determined by an expert as to which of view directions will be used to train the deep reinforcement learning agent, or whether it is to be trained with only one view direction, or trained with two or more view directions, and the like.

The single-stage DRL application unit 140 may use the deep reinforcement learning agent to detect a feature point corresponding to the landmark point on 2D view image which is constructed by the 3D volume rendered model data. The feature point detected in the 2D view image corresponds to two coordinate values of the 3D coordinate values of the landmark point.

In addition, the single-stage DRL application unit 140 may use 1D profile of the 3D image data (CT data) obtained based on the 2D coordinate values previously detected in the 2D view image, to obtain the remaining one coordinate value corresponding to the landmark point and finally detect the landmark point. In this case, the 1D profile of the 3D image data is constructed of 3D image data values on an axis perpendicular to the 2D view image and passing through the feature point.

For example, as illustrated in the upper part of INFERENCING phase 2 and phase 3 of FIG. 2, when the landmark is bregma, the single-stage DRL application unit 140 may first detect the 2D coordinate values (x, y) of the feature point corresponding to the landmark in the 2D top view image using the deep reinforcement learning agent. In addition, the single-stage DRL application unit 140 applies the first order derivative-based boundary estimation technique to the 1D profile of the 3D image data to obtain the remaining one coordinate value (z) of the 3D coordinate values of the landmark. In this case, the 1D profile of the 3D image data represents a 3D image data value on an axis perpendicular to the 2D view image and passing through the feature point (x, y).

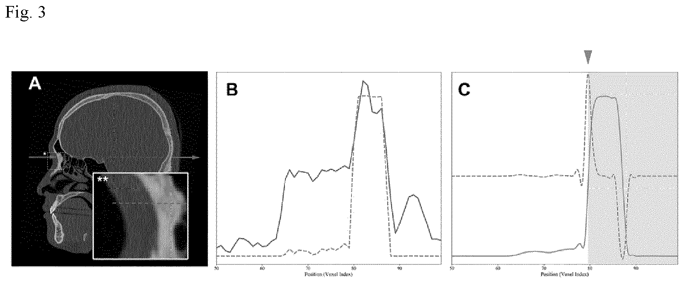

FIG. 3 is a diagram provided to explain a first order derivative-based boundary estimation technique according to an embodiment of the present invention.

Referring to FIG. 3, a result of sharp gradient change of the CT value (3D image data value) at the boundary between the soft tissue and the cortical bone (see FIG. 3A) may be used to detect the depth of the landmark. For example, in order to obtain a landmark (such as a nasion point) on the surface of bone, the DRL algorithm may be applied to the anterior view of the skull to obtain x and z values of the 3D coordinates of the landmark. The 1D profile of the other CT value may be obtained along the y-axis from the feature point (x, z) through robust boundary detection using the slope values proposed below. The bone strength enhancement function IE(x) may be defined as in Equation 1 below.

[Equation 1]

$$IE(x) = \tanh\left(\frac{x - L}{W}\right)$$

where x is the CT value, L is the center of the CT value range, and W is the scale value. Applying IE(x) results in transformation of the 1D profile of CT value (blue line in FIG. 3A) into a simple profile with enhanced bone strength (orange line in FIG. 3B). However, in the presence of noise, it is necessary to remove the noise in boundary detection through the first order derivative. To this end, a nonlinear diffusion equation using structure tensor may be applied. After taking the first order derivative of the noise-reduced profile, the remaining coordinate values may be determined by having the position having the maximum value as the position of the bone surface (see FIG. 3C).

FIG. 3A illustrates a cross-sectional CT image of the sagittal plane, showing a radiation beam mimic line (dashed line) passing through the detected landmark, the nasion, and a box indicated by an asterisk (*). The box indicated by an asterisk (*) is enlarged and shown in a larger box in the lower left side having double asterisk (**) and y-direction line (dashed line). The solid line in FIG. 3B is ID graph of the image density profile of the Hounsfield unit value along the y-axis line (corresponding to the dashed line in FIG. 3A) passing through air, nasion, soft tissue, and bone tissue. In addition, the dashed line in FIG. 3B represents the bone intensity-enhanced profile. In FIG. 3C, the solid line represents the nonlinear diffusion profile of the bone intensity-enhanced profile, and the final boundary estimation of the bone is indicated with the arrowhead and the gray area.

The multi-stage DRL application unit 150 may detect a landmark point by repeating the operation of obtaining 2D coordinate value corresponding to the landmark point on 2D view image, which is constructed by the 3D volume rendered model data, by using the deep reinforcement learning agent.

Specifically, the multi-stage DRL application unit 150 may primarily extract the feature point corresponding to the landmark point on the 2D projection view image by using the deep reinforcement learning agent. In addition, the multi-stage DRL application unit 150 may re-extract the feature point corresponding to the landmark point by using the deep reinforcement learning agent, from a 2D cut plane view image which is newly constructed by using the 2D coordinate values of the feature point obtained from the 2D projection view image.

For example, as exemplified in the lower part of INFERENCING phase 2 and phase 3 of FIG. 2, for porion, first, the coordinate values y and z of the feature point may be obtained from the 2D side view image by using the first deep reinforcement learning agent. In addition, based on the previously determined y-coordinate value, a 2D cut plane view image may be constructed, and the coordinate values x and z of the feature point may be obtained by using the second deep reinforcement learning agent. By using the coordinate values y obtained using the first deep reinforcement learning agent, and the coordinate values x and z obtained using the second deep reinforcement learning agent, the coordinate values x, y, and z of the landmark can be finally obtained.

It is needless say that, depending on embodiments, it may be possible to obtain the coordinate values y and z of the feature point by using the first deep reinforcement learning agent, construct the 2D cut plane view image based on the previously determined z coordinate value, and obtain the coordinate values x and y of the feature point by using the second deep reinforcement learning agent. As described above, regarding which direction of 2D projection view, the 2D cut view will be used for the first and second detections, this may be previously determined according to the target landmark of detection.

According to an embodiment, it may be implemented such that, depending on the type of the landmark, the operation of obtaining the 2D coordinate value corresponding to the landmark by using the deep reinforcement learning agent is repeated three or more times. For example, it is possible to repeat the operation of newly constructing a 2D cut plane view image by using one of the coordinate values x and z obtained using the second deep reinforcement learning agent described above, and detecting the feature point by using the deep reinforcement learning agent. For certain landmark such as the infraorbital foramen, it is possible to repeat DRL three or more times to finally detect the landmark.

The storage unit 180 may store various data, programs, and the like related to the operation of the 3D landmark detection device 100.

The storage unit 180 may be implemented as a magnetic medium such as hard disk, floppy disk, and magnetic tape, an optical medium such as CD-ROM or DVD, a magneto-optical medium such as floptical disk, and a memory device such as ROM, RAM, flash memory, and the like.

The display unit 185 may display on the screen various types of information related to the operation of the 3D landmark detection device 100. The display unit 185 may be implemented as a display device such as LCD display device, LED display device, and the like.

The control unit 190 may control the overall operation of the 3D landmark detection device 100. The control unit 190 detects a landmark by selectively applying one of the single-stage DRL and multi-stage DRL according to the type of landmark.

The type of landmark may be previously determined by an expert. Depending on an embodiment, it may be automatically classified by a learned classifier or changed.

In addition, whether to apply the single-stage DRL or the multi-stage DRL may be predetermined for each type of landmark. For those types for which sufficient accuracy is ensured with the single-stage DRL only, it may be determined to apply the single-stage DRL to avoid computational burden and complexity, and otherwise, it may be determined to apply the multi-stage DRL.

The type of landmark may be classified by anatomical or geometrical characteristics. In general, the type of landmark may be broadly classified into three types as follows.

Type 1 may include bregma and nasion, and the landmark point may be a juxtaposition position of two or more bone tissues.

Type 2 may include sella, anterior nasal spine, infraorbital foramen, orbital, mental foramen, and the like, and may be a landmark point on the maxima of curvature or local morphogenetic processes.

Type 3 may include porion, center of foramen magnum, mandibular foramen, and pogonion.

For the types of landmark exemplarily classified above, whether to apply the single-stage DRL or the multi-stage DRL may be previously determined for each landmark. This may be determined differently according to the embodiment or by the operator using the device. However, the multi-stage DRL may be applied to landmarks that do not have a landmark on the bone surface and where it is not possible to specify a corresponding landmark in the surrounding bone.

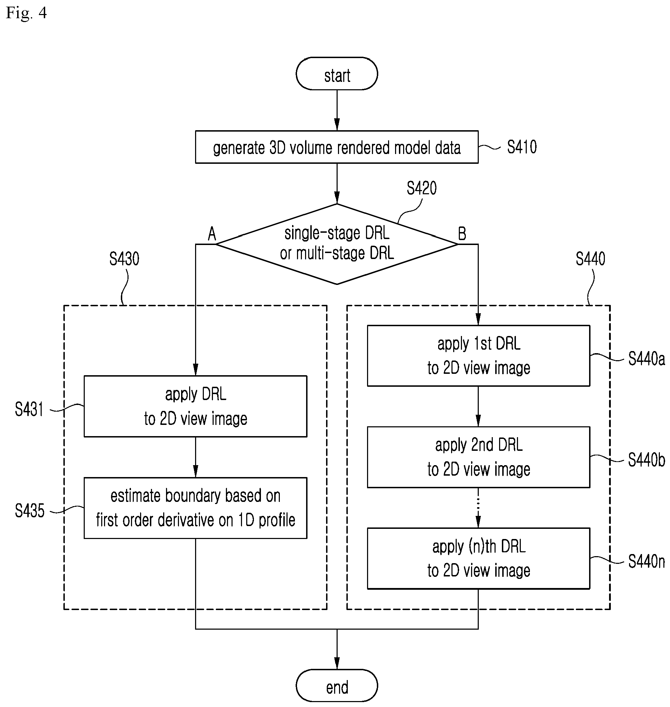

FIG. 4 is a flowchart illustrating a 3D anatomical landmark detection method using multi-stage deep reinforcement learning according to an embodiment of the present invention.

Referring to FIG. 4, first, the 3D volume rendered model generation unit 120 may perform volume rendering on 3D image data to generate 3D volume rendered model data (S410).

Then, the control unit 190 selects whether to apply the single-stage DRL or the multi-stage DRL according to the type of the target landmark of detection (S420).

When it is selected to apply the single-stage DRL (S420-A), the single-stage DRL application unit 140 may use the deep reinforcement learning agent to detect a feature point corresponding to the landmark point on 2D view image which is constructed by the 3D volume rendered model data (S431). The feature point detected in the 2D view image corresponds to two coordinate values of the 3D coordinate values of the landmark point.

In addition, the single-stage DRL application unit 140 may apply the first order derivative-based boundary estimation technique to the 1D profile of the 3D image data (CT data) obtained based on the 2D coordinate values previously detected in the 2D view image, to obtain the remaining one coordinate value corresponding to the landmark point and finally detect the landmark point (S435). In this case, the 1D profile of the 3D image data is constructed of 3D image data values on an axis perpendicular to the 2D view image and passing through the feature point.

Meanwhile, when it is selected to apply the multi-stage DRL (S420-B), the multi-stage DRL application unit 150 may use the first deep reinforcement learning agent to primarily extract the feature point corresponding to the landmark point on the 2D projection view image (S440a).

In addition, the multi-stage DRL application unit 150 may use the second deep reinforcement learning agent to re-extract the feature point corresponding to the landmark point on a 2D cut plane view image which is newly constructed by using the 2D coordinate values of the feature point obtained from the 2D projection view image (S440b). As such, the multi-stage DRL application unit 150 may detect the 3D coordinate values of the landmark by using the two coordinate values of the feature point obtained in S440a and the one coordinate value obtained in S440a.

For example, it is assumed that the coordinate values of the feature point obtained in S440a from the predetermined 2D view image (xy plane) by applying the primary DRL to the target landmark are $(x_1, yi)$, and that the plane determined to apply the secondary DRL to the landmark is $y = y_1$. In addition, when the coordinate values of the feature point obtained from the 2D cut view image corresponding to the plane $(y = y_1)$ by applying the secondary DRL are $(x_2, z_2)$, the 3D coordinate values of the landmark may be detected as, most simply, $(x_2, y_1, z_2)$.

Meanwhile, according to an embodiment, it may be implemented such that, depending on the type of the landmark, the operation of obtaining the 2D coordinate value corresponding to the landmark by using the deep reinforcement learning agent is repeated from three to n times (S440n). In this case, it is possible to obtain the 3D coordinate value of the landmark by using the 2D coordinate value of the feature point obtained by using the last deep reinforcement learning agent and one coordinate value of the remaining dimension obtained immediately before it.

The embodiments described above may be implemented as a hardware component, a software component, and/or a combination of a hardware component and a software component. For example, the devices, methods, and components described in the embodiments may be implemented by using one or more general computer or specific-purpose computer such as a processor, a controller, an arithmetic logic unit (ALU), a digital signal processor, a microcomputer, a field programmable gate array (FPGA), a programmable logic unit (PLU), a microprocessor, or any other device capable of executing instructions and responding thereto. The processing device may execute an operating system (OS) and one or more software applications executed on the operating system. Further, the processing device may access, store, operate, process, and generate data in response to the execution of software. For convenience of understanding, although it is described in certain examples that one processing device is used, one of ordinary skill in the art may understand that the processing device may include a plurality of processing elements and/or a plurality of types of processing elements. For example, the processing device may include a plurality of processors or one processor and one controller. In addition, other processing configurations such as a parallel processor are possible.

The software may include a computer program, code, instructions, or a combination of one or more of the above, and may configure the processing unit, or instruct the processing unit independently or collectively to operate as desired. Software and/or data may be interpreted by the processing device or, in order to provide instructions or data to the processing device, may be embodied in any type of machine, component, physical device, virtual equipment, or computer storage medium or device, permanently or temporarily. The software may be distributed over networked computer systems and stored or executed in a distributed manner. The software and data may be stored on one or more computer-readable recording media.

The method according to the embodiments may be implemented in the form of program instructions that can be executed through various computer means and recorded in a computer-readable medium. The computer readable medium may include program instructions, data files, data structures, and the like alone or in combination. The program instructions recorded on the medium may be those specially designed and configured for the purposes of the embodiments, or may be known and available to those skilled in computer software. Examples of computer readable recording medium include magnetic media such as hard disks, floppy disks, and magnetic tape, optical media such as CD-ROMs and DVDs, magneto-optical media such as floptical disks, and hardware devices specifically configured to store and execute program instructions such as ROM, RAM, flash memory, and the like. Examples of the program instructions include machine language codes such as those generated by a compiler, as well as high-level language codes that may be executed by a computer using an interpreter, and so on. The hardware device described above may be configured to operate as one or more software modules in order to perform the operations according to the embodiments, and vice versa.

As described above, although the embodiments have been described with reference to the limited drawings, a person of ordinary skill in the art can apply various technical modifications and variations based on the above. For example, even when the described techniques are performed in the order different from the method described above, and/or even when the components of the system, structure, device, circuit, and the like are coupled or combined in a form different from the way described above, or replaced or substituted by other components or equivalents, an appropriate result can be achieved.

**Claims**

1. A 3D landmark detection method comprising:

   performing volume rendering on 3D image data to generate 3D volume rendered model data; and
   applying a single-stage deep reinforcement learning (DRL), wherein
   the applying the single-stage DRL comprises:

      obtaining a 2D coordinate value corresponding to a landmark point on a 2D view image, which is constructed by the 3D volume rendered model data, by using a deep reinforcement learning agent; and
      obtaining a remaining 1D coordinate value corresponding to the landmark point based on an 1D profile of the 3D image data which is obtained based on the 2D coordinate value, wherein the 1D profile is constructed of a 3D image data value on an axis perpendicular to the 2D view image and passing through a point that corresponds to the 2D coordinate value.

2. The 3D landmark detection method according to claim 1, further comprising applying a multi-stage DRL, wherein the applying the multi-stage DRL comprises detecting the landmark point by repeating the operations of:

      obtaining the 2D coordinate value corresponding to the landmark point on the 2D view image, which is constructed by the 3D volume rendered model data, by using the deep reinforcement learning agent; and
      from a 2D view image which is determined based on the obtained 2D coordinate value, obtaining a 2D coordinate value corresponding to the landmark point by using a deep reinforcement learning agent, wherein
      the applying the single-stage DRL or the applying the multi-stage DRL is selectively employed according to characteristics of the landmark point.

3. The 3D landmark detection method according to claim 2, wherein the deep reinforcement learning agent is trained with a deep reinforcement learning algorithm to detect a feature point corresponding to the landmark point on the 2D view image which is constructed by the 3D volume rendered model data.

4. The 3D landmark detection method according to claim 3, wherein the applying the single-stage DRL includes:

> obtaining the 2D coordinate value corresponding to the landmark point on the 2D view image, which is constructed by the 3D volume rendered model data, through the deep reinforcement learning agent; and
> obtaining the remaining one coordinate value based on a boundary point detected by using a first order derivative value in the 1D profile of the 3D image data corresponding to the remaining 1D coordinate value, or
> the applying the single-stage DRL comprises:
>
> > obtaining the 2D coordinate value corresponding to the landmark point on the 2D view image, which is constructed by the 3D volume rendered model data, through the deep reinforcement learning agent;
> > transforming the 1D profile of the 3D image data corresponding to the remaining 1D coordinate value by using the following equation; and
> > applying a nonlinear diffusion equation to the transformed profile to take a first order derivative of a profile with reduced noise, and then obtaining the remaining 1D coordinate value based on a position with a maximum slope,

$$ IE(x) = \tanh\left(\frac{x - L}{W}\right) $$

> > where x is the 3D image data value, L is a center of a 3D image data value range, and W is a scale value.

5. The 3D landmark detection method according to claim 3, wherein the applying the multi-stage DRL comprises:

> obtaining a first 2D coordinate value corresponding to the landmark point on a 2D projection view image, which is constructed by the 3D volume rendered model data, by using a first deep reinforcement learning agent; and obtaining a second 2D coordinate value corresponding to the landmark point on a first cut view image, which is constructed by the 3D volume rendered model data, by using a second deep reinforcement learning agent, wherein the first cut view image corresponds to a plane defined by the first 2D coordinate value.

6. The 3D landmark detection method according to claim 5, comprising obtaining a 3D coordinate value corresponding to the landmark point by using a coordinate value among the first 2D coordinate values which is not included in the second 2D coordinate value, and the second 2D coordinate value.

7. The 3D landmark detection method according to claim 5, wherein the applying the multi-stage DRL further comprises obtaining a third 2D coordinate value corresponding to the landmark point on a second cut view image, which is constructed by the 3D volume rendered model data, by using a third deep reinforcement learning agent, wherein the second cut view image corresponds to a plane defined by the second 2D coordinate value.

8. The 3D landmark detection method according to claim 3, wherein:

> the 3D image data is acquired by capturing a patient's skull or other parts of a body, and is any one of Computed Tomography (CT), Cone Beam Computed Tomography (CBCT), Magnetic Resonance Imaging (MRI), Positron Emission Tomography (PET), and Single-Photon Emission Computed Tomography (SPECT); and
> the characteristics of the landmark are previously determined.

9. A computer-readable recording medium comprising:

> a set of instructions for performing volume rendering on 3D image data to generate 3D volume rendered model data; and
> a set of instructions for applying a single-stage deep reinforcement learning (DRL), wherein the applying the single-stage DRL comprises:
>
> > obtaining a 2D coordinate value corresponding to a landmark point on a 2D view image, which is constructed by the 3D volume rendered model data, by using a deep reinforcement learning agent; and
> > obtaining a remaining 1D coordinate value corresponding to the landmark point based on an 1D profile of the 3D image data which is obtained based on the 2D coordinate value, wherein the 1D profile is constructed of a 3D image data value on an axis perpendicular to the 2D view image and passing through a point that corresponds to the 2D coordinate value.

10. The computer-readable recording medium according to claim 9, further comprising:

a set of instructions for applying a multi-stage DRL, wherein the applying the multi-stage DRL comprises detecting the landmark point by repeating the operations of:

obtaining the 2D coordinate value corresponding to the landmark point on the 2D view image, which is constructed by the 3D volume rendered model data, by using the deep reinforcement learning agent; and from a 2D view image which is determined based on the obtained 2D coordinate value, obtaining a 2D coordinate value corresponding to the landmark point by using a deep reinforcement learning agent, wherein the applying the single-stage DRL or the applying the multi-stage DRL is selectively applied according to characteristics of the landmark point.

11. A 3D landmark detection device comprising:

a 3D volume rendered model generation unit configured to perform volume rendering on 3D image data to generate 3D volume rendered model data; and a single-stage DRL application unit configured to obtain a 2D coordinate value corresponding to a landmark point on a 2D view image, which is constructed by the 3D volume rendered model data, by using a deep reinforcement learning agent, and obtain a remaining 1D coordinate value corresponding to the landmark point based on an 1D profile of the 3D image data which is obtained based on the 2D coordinate value, wherein the 1D profile is constructed of a 3D image data value on an axis perpendicular to the 2D view image and passing through a point that corresponds to the 2D coordinate value.

12. The 3D landmark detection device according to claim 11, further comprising:

a multi-stage DRL application unit configured to detect the landmark point by repeating the operations of:

obtaining the 2D coordinate value corresponding to the landmark point on the 2D view image, which is constructed by the 3D volume rendered model data, by using the deep reinforcement learning agent;, and from a 2D view image which is determined based on the obtained 2D coordinate value, obtaining a 2D coordinate value corresponding to the landmark point by using a deep reinforcement learning agent, wherein the applying the single-stage DRL or the applying the multi-stage DRL is selectively applied according to characteristics of the landmark point.

13. The 3D landmark detection device according to claim 12, wherein the deep reinforcement learning agent is trained with a deep reinforcement learning algorithm to detect a feature point corresponding to the landmark point on the 2D view image which is constructed by the 3D volume rendered model data.

14. The 3D landmark detection device according to claim 13, wherein the single-stage DRL application unit is configured to:

obtain the 2D coordinate value corresponding to the landmark point on the 2D view image, which is constructed by the 3D volume rendered model data, through the deep reinforcement learning agent; and obtain the remaining one coordinate value based on a boundary point detected by using a first order derivative value in the 1D profile of the 3D image data corresponding to the remaining 1D coordinate value, or wherein the single-stage DRL application unit is configured to:

obtain the 2D coordinate value corresponding to the landmark point on the 2D view image, which is constructed by the 3D volume rendered model data, through the deep reinforcement learning agent; transform the 1D profile of the 3D image data corresponding to the remaining 1D coordinate value by using the following equation; and apply a nonlinear diffusion equation to the transformed profile to take a first order derivative of a profile with reduced noise, and then obtain the remaining 1D coordinate value based on a position with a maximum slope,

$$IE(x) = \tanh\left(\frac{x - L}{W}\right)$$

where x is the 3D image data value, L is a center of a 3D image data value range, and W is a scale value.

**15.** The 3D landmark detection device according to claim 13, wherein the multi-stage DRL application unit is configured to:

obtain a first 2D coordinate value corresponding to the landmark point on a 2D projection view image, which is constructed by the 3D volume rendered model data, by using a first deep reinforcement learning agent; and
obtain a second 2D coordinate value corresponding to the landmark point on a first cut view image, which is constructed by the 3D volume rendered model data, by using a second deep reinforcement learning agent, wherein
the first cut view image corresponds to a plane defined by the first 2D coordinate value.

Fig. 1

Fig. 2

Fig. 3

Fig. 4

```
                        ┌──────────┐
                        │  start   │
                        └────┬─────┘
                             │
                             ▼
        ┌────────────────────────────────────────┐
        │  generate 3D volume rendered model data │ ～ S410
        └────────────────────┬───────────────────┘
                             │                      S420
                             ▼                     ╱
              A         ◇◇◇◇◇◇◇◇◇◇◇◇◇◇◇◇         B
        ┌────────────◇   single-stage DRL   ◇────────────┐
   S430 │            ◇   or multi-stage DRL  ◇            │ S440
        │             ◇◇◇◇◇◇◇◇◇◇◇◇◇◇◇◇             │
   ┌─ ─ ┼─ ─ ─ ─ ─ ─ ─ ─ ─┐     ┌─ ─ ─┼─ ─ ─ ─ ─ ─ ─ ─ ─┐
   │    │                  │     │     ▼                  │
   │    │                  │     │ ┌──────────────────┐   │
   │    │                  │     │ │   apply 1st DRL   │ ─ S440a
   │    │                  │     │ │  to 2D view image │   │
   │    ▼                  │     │ └─────────┬────────┘   │
   │ ┌──────────────────┐  │     │           │            │
 S431 │    apply DRL     │  │     │           ▼            │
   │ │  to 2D view image │  │     │ ┌──────────────────┐   │
   │ └─────────┬────────┘  │     │ │   apply 2nd DRL   │ ─ S440b
   │           │           │     │ │  to 2D view image │   │
   │           ▼           │     │ └─────────┬────────┘   │
   │ ┌──────────────────┐  │     │           ┊            │
 S435 │ estimate boundary │  │     │           ▼            │
   │ │based on first order│  │     │ ┌──────────────────┐   │
   │ │derivative on 1D    │  │     │ │   apply (n)th DRL │ ─ S440n
   │ │profile            │  │     │ │  to 2D view image │   │
   │ └─────────┬────────┘  │     │ └─────────┬────────┘   │
   └─ ─ ─ ─ ─ ─┼─ ─ ─ ─ ─ ─┘     └─ ─ ─ ─ ─ ─┼─ ─ ─ ─ ─ ─┘
               └───────────┬─────────────────┘
                           ▼
                      ┌─────────┐
                      │   end   │
                      └─────────┘
```

Europäisches Patentamt

European Patent Office

Office européen des brevets

# EUROPEAN SEARCH REPORT

Application Number

**EP 21 21 4467**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KUNDETI SRINIVASA RAO ET AL: "Landmark Detection in 3D Medical Images Using Reinforcement Learning", 2020 IEEE INTERNATIONAL CONFERENCE ON CLOUD COMPUTING IN EMERGING MARKETS (CCEM), IEEE, 6 November 2020 (2020-11-06), pages 42-46, XP033952921, DOI: 10.1109/CCEM50674.2020.00019 [retrieved on 2021-07-27] * the whole document * | 1-15 | INV. G06V10/44 |
| A | FLORIN-CRISTIAN GHESU ET AL: "Multi-Scale Deep Reinforcement Learning for Real-Time 3D-Landmark Detection in CT Scans", IEEE TRANSACTIONS ON PATTERN ANALYSIS AND MACHINE INTELLIGENCE, vol. 41, no. 1, 11 December 2017 (2017-12-11), pages 176-189, XP055725456, USA ISSN: 0162-8828, DOI: 10.1109/TPAMI.2017.2782687 * the whole document * | 1-15 | |
| | | | TECHNICAL FIELDS SEARCHED (IPC) |
| | | | G06V |

The present search report has been drawn up for all claims

| Place of search | Date of completion of the search | Examiner |
|---|---|---|
| The Hague | 6 May 2022 | Isa, Sabine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)